# FASCICULE DE BREVET EUROPEEN

(11) **EP 1 928 447 B1**
(45) Date de publication et mention de la délivrance du brevet: **17.02.2016**
(21) Numéro de dépôt: 06764763.6
(22) Date de dépôt: 09.06.2006
(51) Int. Cl.: A61K 31/192, A61K 8/36, A61K 36/898

(54) **EXTRAIT DE VANILLA PLANIFOLIA, SON PROCEDE D' OBTENTION, ET COMPOSITION COSMETIQUE OU DERMATOLOGIQUE LE CONTENANT**
EXTRAKT AUS VANILLA PLANIFOLIA, VERFAHREN ZU SEINER GEWINNUNG UND DIESEN ENTHALTENDE KOSMETISCHE ODER DERMATOLOGISCHE ZUSAMMENSETZUNG
VANILLA PLANIFOLIA EXTRACT, METHOD FOR OBTAINING SAME, AND COSMETIC OR DERMATOLOGICAL COMPOSITION CONTAINING SAME

(30) Priorité: 23.09.2005 FR 0509765; 23.09.2005 FR 0509767
(43) Date de publication de la demande: 11.06.2008
(73) Titulaire: Chanel Parfums Beauté, 92521 Neuilly sur Seine Cedex (FR)
(72) Inventeur: MAESTRO, Yannick, F-13500 Martigues (FR); BERGIA, Daniel, F-06560 Valbonne (FR); LASSERRE, Christelle, Jersey City, NJ 07302 (US)
(74) Mandataire: Cabinet Plasseraud
(86) Numéro de dépôt international: PCT/FR2006/001312
(87) Numéro de publication internationale: WO 2007/034042

(56) Documents cités:
- WO-A-2004/084855
- FR-A- 2 073 240
- FR-A- 2 837 384
- LAMPRECHT G ET AL: "Determination of the authenticity of vanilla extracts by stable isotope ratio analysis and component analysis by HPLC" JOURNAL OF AGRICULTURAL AND FOOD CHEMISTRY, AMERICAN CHEMICAL SOCIETY. WASHINGTON, US, vol. 42, 1994, pages 1722-1727, XP002218297 ISSN: 0021-8561
- RANADIVE A S: "Vanillin and related flavor compounds in vanilla extracts made from beans of various global origins" JOURNAL OF AGRICULTURAL AND FOOD CHEMISTRY, AMERICAN CHEMICAL SOCIETY. WASHINGTON, US, vol. 40, 1992, pages 1922-1924, XP002218298 ISSN: 0021-8561

## Description

L'invention concerne un nouvel extrait de *Vanilla planifolia,* constitué d'une fraction liposoluble, son procédé d'obtention, une composition cosmétique ou dermatologique le contenant, ainsi que son utilisation en tant qu'agent actif polyfonctionnel dans une composition cosmétique ou dermatologique, pour la prévention et/ou le traitement d'altérations de la peau dus, notamment, au vieillissement ou à des mécanismes physiologiques liés au vieillissement ou à des troubles apparentés à ces mécanismes.

La peau est principalement constituée de trois couches, à savoir, en partant de la plus superficielle, l'épiderme, le derme et l'hypoderme.

L'épiderme contribue largement à assurer la protection de la peau et à en maintenir la trophicité.

Le vieillissement et le photoviellissement de la peau et les altérations qui y sont associées peuvent se manifester de différentes manières, parmi lesquelles on peut citer :
- la perte de fermeté et d'élasticité dues à une perte tissulaire au niveau de l'épiderme et/ou du derme;
- la perte d'éclat due à la réduction de la microcirculation et à un ralentissement du renouvellement cellulaire au niveau de l'épiderme ;
- l'apparition de taches pigmentaires associées à un dysfonctionnement de la synthèse de la mélanine (ou mélanogénèse) ;
- la sécheresse cutanée résultant d'une diminution de la fonction barrière de la couche cornée et à un ralentissement du renouvellement épidermique.

Il existe, de ce fait, un besoin de fournir un agent actif polyfonctionnel susceptible d'agir sur un ensemble de causes d'altérations de la peau dues au vieillissement et/ou à une modification des mécanismes physiologiques liés au vieillissement ou apparentés.

La demande FR 2 837 384 décrit l'utilisation d'extrait de vanille, de préférence de *Vanilla tahitensis*, pour la préparation de compositions cosmétiques ou pharmaceutiques pour la protection de la peau contre les radiations solaires ou contre la génération de radicaux libres, ces activités étant liées à la présence de polyphénols contenus dans cet extrait.

On a maintenant trouvé qu'un extrait de *Vanilla planifolia,* constitué d'une fraction liposoluble, présentait, par le biais de stimulation ou d'inhibition de mécanismes physiologiques, des activités susceptibles d'agir sur des symptômes dus au vieillissement, ou à des mécanismes physiologiques liés au vieillissement, ou à des troubles apparentés à ces mécanismes au niveau de l'épiderme et/ou du derme.

De manière surprenante, ces activités ne sont pas liées à la présence de polyphénols dans cet extrait.

L'invention concerne donc, selon un premier aspect, un extrait de *Vanilla planifolia,* constitué d'une fraction liposoluble selon la revendication 1. De préférence ladite fraction comprend :
- 0,5 à 10% de composés monocarbonylés insaturés,
- 20 à 80% de composés dicarbonylés insaturés, et
- 1 à 40% de pyranones insaturées,
lesdites teneurs étant exprimées en pourcentages relatifs par rapport à l'ensemble des constituants séparés par chromatographie en phase gazeuse.

Par « fraction liposoluble », on entend la fraction qui, lorsqu'on effectue une extraction à l'aide d'un solvant, après broyage et/ou macération de la vanille, suivie ou non d'une décantation à l'aide d'un solvant organique entraînant une séparation de phases, est soluble dans une phase huileuse et non dans une phase aqueuse. Il s'agit plus particulièrement d'une fraction insoluble dans l'eau à raison d'au moins 1% en poids à 25°C et soluble dans les mêmes conditions dans au moins un solvant apolaire ayant un indice de polarité inférieur à 3.5, notamment choisi parmi : l'hexane, le cyclohexane, l'heptane, l'isooctane et le dichlorométhane. Le solvant apolaire a de préférence un indice de polarité inférieur à 1 et est donc de préférence choisi parmi : l'hexane, le cyclohexane, l'heptane et l'isooctane.

Par « teneur exprimée en pourcentages relatifs par rapport à l'ensemble des constituants séparés par chromatographie en phase gazeuse », on entend que la teneur en chacun des constituants est déterminée par rapport à l'ensemble des constituants séparés par le système chromatographique. Seuls sont présents les composés extraits par le solvant lors de la préparation de l'échantillon et pouvant se vaporiser dans l'injecteur.

L'échantillon est de préférence préparé selon la norme NF T 60-233 de mai 1977 « Préparation des esters méthyliques d'acides gras », (§ 5.2 - Méthode applicable aux corps gras acides et acides gras. Les conditions chromatographiques sont décrites dans la norme NF EN ISO 5508 de juin 1995.

Brièvement, la méthode consiste à estérifier les acides gras en milieu méthanolique acide, puis à les extraire avec de l'heptane, puis à injecter la solution heptanique en chromatographie en phase gazeuse.

De préférence, l'extrait de *Vanilla planifolia* selon l'invention est constitué d'une fraction liposoluble comprenant :
- 1 à 5% de composés monocarbonylés insaturés,
- 35 à 65% de composés dicarbonylés insaturés, et
- 3 à 25% de pyranones insaturées,
   lesdites proportions étant exprimées en pourcentages relatifs par rapport à l'ensemble des constituants séparés par chromatographie en phase gazeuse.

Selon des aspects préférés de l'invention, ledit extrait comprend
- des composés monocarbonylés insaturés choisis parmi la Pentacosén-16-one-2, la Heptacosén-18-one-2 et la Nonacosén-20-one-2 et/ou
- des composés dicarbonylés insaturés choisis parmi la Pentacosène-16-dione-2,4, la Heptacosène-18-dione-2,4, la Nonacosène-20-dione-2,4 et l' Hentriacontène-22-dione-2,4 et/ou
- des pyranones insaturées choisies parmi la nonadécényl-2 dihydro-2,3 méthyl-6 pyranone-4, l'hénéicosényl-2 dihydro-2,3 méthyl-6 pyranone-4 et la tricosényl-2 dihydro-2,3 méthyl-6 pyranone-4.

En particulier, ladite fraction liposoluble comprend, en outre, 5 à 50%, de préférence 10 à 40% d'acides gras saturés ou insaturés, lesdites proportions étant exprimées en pourcentages relatifs par rapport à l'ensemble des constituants séparés par chromatographie en phase gazeuse.

Ledit extrait comprend également, selon un aspect préféré, des composés stérols choisis parmi le cholestérol, le campestérol, le stigmastérol et le β-sitostérol.

Il peut en variante ou en plus comprendre des dérivés (notamment hydroxylés ou alcoxylés) de benzaldéhyde et en particulier le 4-hydroxy-3-méthoxy-benzaldéhyde ou vanilline, de préférence en quantité de 0.1 à 10% en poids.

La matière première mise en oeuvre consiste, par exemple, en des gousses de vanille de l'espèce *Vanilla planifolia,* que l'on peut broyer ou réduire en morceaux de manière usuelle.

Le broyat peut être soumis à une extraction par un ou plusieurs solvants, par exemple choisis parmi : les alcools en C₁-C₄ tels que par exemple le méthanol, l'éthanol, l'isopropanol, etc., les solvants organiques tels que par exemple le propylène glycol, le dipropylène glycol etc., ou encore l'acétate d'éthyle, l'hexane, le cyclohexane ou tout autre solvant organique usuel dans le domaine, ou encore par fluide supercritique, de préférence par CO₂ supercritique. Les solvants alcooliques sont préférés.

L'extraction est généralement réalisée en immergeant ou en agitant doucement le broyat dans un ou plusieurs des solvants cités ci-dessus à des températures allant, par exemple, de la température ambiante à 100°C, pendant une durée d'environ 30 min à 12 h.

La solution est alors filtrée afin d'éliminer les substances insolubles de la plante. On élimine également, le cas échéant, le solvant s'il s'agit d'un solvant volatil tel que, par exemple, l'éthanol, le méthanol, l'hexane, le cyclohexane ou l'acétate d'éthyle.

Cette étape d'extraction est usuelle dans le domaine des extraits de plantes, et l'homme du métier est à même d'en ajuster les paramètres réactionnels, sur la base de ses connaissances générales.

A l'issue de cette étape d'extraction, on obtient une oléorésine de vanille.

Selon un aspect avantageux de l'invention, on effectue une étape de fractionnement afin de purifier l'oléorésine.

On utilisera, de préférence, un solvant ou un mélange de solvants choisi(s) parmi les solvants cités ci-dessus, ou de préférence un solvant hydro-alcoolique. Un fractionnement par fluide supercritique, de préférence par CO₂ supercritique, peut également être utilisé.

Comme précédemment, si un ou plusieurs solvants volatils est (sont) utilisé(s), il y aura lieu de le ou les éliminer avant de passer à l'étape suivante.

On récupère la fraction liposoluble provenant, soit de l'extraction, en ayant utilisé un solvant d'extraction approprié, soit, après décantation, de l'étape de fractionnement mentionnée ci-dessus.

Ladite fraction liposoluble peut être utilisée comme extrait de *Vanilla planifolia* selon l'invention.

Ladite fraction liposoluble est ensuite soumise à une étape de distillation moléculaire.

Les distillateurs moléculaires à film raclé et à court trajet sont préférés. Ils comprennent une chambre de distillation dotée d'un racleur tournant, permettant l'étalement en continu sur la surface d'évaporation (surface chaude) de produit à distiller. Les vapeurs de produit sont condensées par le biais d'un doigt réfrigéré, placé au centre de la chambre de distillation. La récupération des résidus et distillat se fait par écoulement gravitationnel. Cette technique a pour objectif de séparer les constituants des mélanges complexes en tirant profit de leurs différents points d'ébullition. La distillation moléculaire à film raclé et à court trajet a pour avantage de réduire la température de distillation car la distillation s'effectue sous un vide poussé et de réduire également le temps de séjour du mélange à séparer dans le distillateur. En effet, la vitesse de décomposition des produits s'accroît énormément en fonction de la température et du temps d'exposition et dans un distillateur de type « alambic » par exemple, les mélanges peuvent séjourner pendant des heures à des températures importantes, ce qui entraîne une dénaturation.

Jusqu'à maintenant, dans le cas des huiles végétales, un tel procédé n'avait été utilisé que pour isoler des fractions insaponifiables ou purifier ces mêmes huiles végétales en éliminant les fractions insaponifiables, ou encore, dans le cas de fractions hydrosolubles ou liposolubles, pour décolorer ou désodoriser les extraits.

Or, on a maintenant trouvé que, de manière surprenante, l'utilisation d'une étape de distillation moléculaire permettait d'isoler un distillat huileux à partir d'une fraction liposoluble telle que décrite précédemment.

Le procédé d'obtention d'un extrait de *Vanilla planifolia* susceptible d'être utilisé comme actif polyfonctionnel selon l'invention comprend en outre une étape de distillation moléculaire de la fraction liposoluble, consistant de préférence à :
- soumettre la fraction liposoluble résultant de l'extraction précitée à une distillation moléculaire à une température comprise entre environ 100 et 250°C et une pression comprise entre environ 0,1 et 0,001 mbar, et
- récupérer le distillat.

Des conditions préférentielles de mise en oeuvre du procédé sont données ci-après :

Avantageusement, on ajoute à la fraction liposoluble, avant la distillation, un solvant lourd choisi parmi les huiles minérales ou végétales et les polyols, tels que de préférence du polyéthylène glycol, pour faciliter la distillation ou l'écoulement le long de la colonne.

Le mélange de la fraction liposoluble et du solvant lourd est introduit à débit constant, à une température comprise entre 20 et 120°C, de préférence entre 50 et 100°C, sur la paroi chaude d'un évaporateur cylindrique.

Par raclage au moyen de balais rotatifs à bagues, il est étalé en film mince sur toute la surface de la paroi chaude maintenue à une température comprise entre environ 100 et 250°C, et, de préférence entre 150 et 220°C.

Au contact de celle-ci, et sous la très faible pression régnant dans l'évaporateur, de l'ordre de 0,1 mbar à 0,001 mbar, le produit volatil est alors partiellement et progressivement vaporisé, alors que le produit moins volatil coule le long de la paroi.

Les vapeurs émises sont condensées sur la paroi froide concentrique de la paroi chaude et placée à très courte distance de celle-ci, de préférence à une température comprise entre 40 et 120°C, notamment entre 60 et 100°C.

Les produits séparés au cours de l'opération s'écoulent par gravité le long des parois chaude et froide.

On récupère le distillat que l'on soumet, le cas échéant, à une opération supplémentaire de séparation (filtration ou centrifugation, par exemple).

On obtient ainsi un distillat huileux qui peut être utilisé comme actif polyfonctionnel selon l'invention.

Avantageusement, l'extrait mis en oeuvre selon l'invention (fraction liposoluble ou distillat huileux) est de couleur claire. De plus, ledit extrait est sensiblement dépourvu, et de préférence totalement exempt, de solvant ou de tout autre réactif chimique étant intervenu au cours de son extraction.

Egalement, ledit extrait se présente sous une forme suffisamment concentrée pour pouvoir être utilisé sans entraîner les problèmes de formulation habituellement rencontrés aux concentrations nécessaires pour obtenir une activité dans les compositions cosmétiques ou dermatologiques sous forme d'émulsion, et sans présenter une couleur foncée, contrairement aux extraits végétaux obtenus par des procédés usuels, lorsqu'ils sont sous forme concentrée.

De ce fait, l'extrait selon l'invention peut être utilisé directement pour la préparation d'une composition cosmétique ou dermatologique.

L'invention concerne également l'extrait de *Vanilla planifolia* susceptible d'être obtenu par le procédé décrit plus haut, c'est-à-dire suivant un procédé comprenant les étapes consistant à :
- procéder à une extraction à partir d'un broyat de *Vanilla planifolia* à l'aide d'au moins un solvant, et
- récupérer la fraction liposoluble,
- et comprenant en outre une étape de distillation moléculaire de la fraction liposoluble pour obtenir un distillat huileux.

Selon un aspect ultérieur, l'utilisation d'un extrait de *Vanilla planifolia* tel que décrit précédemment dans une composition cosmétique, en tant qu'agent améliorant l'atrophie cutanée et/ou agent blanchissant et/ou agent améliorant la microcirculation cutanée et/ou agent de régénération de l'épiderme et/ou agent émollient est décrite.

L'invention concerne également l'utilisation d'un extrait de *Vanilla planifolia* tel que décrit précédemment pour la préparation d'une composition dermatologique dépigmentante.

En effet, de manière avantageuse, on a trouvé que l'extrait de *Vanilla planifolia* selon l'invention possédait plusieurs activités d'intérêt vis-à-vis de mécanismes physiologiques préventifs ou réparateurs liés aux altérations de la peau, dues notamment au vieillissement.

L'invention concerne donc, plus particulièrement, l'utilisation cosmétique d'un extrait de *Vanilla planifolia* selon l'invention en tant qu'agent inhibiteur de la synthèse de mélanine, notamment en tant qu'agent inhibiteur de la synthèse d'endothéline.

On a également trouvé que, de manière avantageuse, l'extrait de *Vanilla planifolia* selon l'invention présentait une activité activatrice à l'égard de la synthèse de plusieurs familles de facteurs de croissance par les kératinocytes.

En particulier, cette activité s'exerce vis-à-vis des facteurs de croissance suivants :
- Le FGF-b (facteur de croissance des fibroblastes basique ou en anglais « basic fibroblast growth factor») également dénommé FGF , b-FGF ou FGF-2, qui joue un rôle important dans la maintenance et la réparation de nombreux tissus, du fait de sa capacité à induire la prolifération cellulaire, notamment celle des fibroblastes, la migration des kératinocytes indispensable lors de la cicatrisation ou lors du vieillissement (Ashcroft GS et al., J. Anat, 1997, 190 (Pt 3):351 -65) ainsi que l'angiogénèse (Bikfalvi et al., Endocrine review, 1997, Vol 18 n°1) ou en réponse aux irradiations UV (Kramer M et al., J. Biol. Chem., 1993, 268(9):6734-41).
   Le FGF-b est sécrété et stocké dans la matrice extracellulaire pour assurer toute réparation tissulaire. De plus, il joue un rôle dans la prolifération et la différenciation des mélanocytes (Tada A et al. Cell, Growth Differ., 1998, 9(7):575-84).
- Le PDGF ou facteur de croissance dérivé des plaquettes (en anglais « platelet-derived growth factor »), qui exerce, notamment, une activité mitogène sur la plupart des cellules dérivées du mésenchyme (Lepisto J et al, 1995, Biochem. Biophys. Res. Commun, 209(2):393-9) et stimule la synthèse de collagène et de collagènase par ces cellules, jouant ainsi un rôle dans des processus physiologiques tels que la cicatrisation et la réparation tissulaire (Tan EM et al, Biochem. J. 1995, 310 (Pt 2):585-8) ; il a également été montré que le niveau d'induction des PDGF et la capacité réplicative des cellules étaient liés à l'âge : des fibroblastes sénescents ont leur taux de PDGF qui diminue (Karlsson C et al., J. Cell Physiol., 1994, 158(2):256-62). Egalement, il a été montré que la quantité et le type des différents facteurs de croissance pouvaient expliquer les différences de capacité des tissus à se réparer avec l'âge. (Ashcroft GS et al., J. Anat, 190, 1997, (Pt 3):351-65).
- Le TGFβ ou facteur de croissance transformant β (en anglais « transforming growth factor β »), qui est une cytokine intervenant dans la régulation de la croissance cellulaire, et qui joue un rôle dans la régulation de la croissance des kératinocytes (Yin L et al., J. Invest. Dermatol., 2003, 120(4):703-5 ; Rittie L et al., Ageing Res. Rev., 2002, 1(4):705-20), et dans la synthèse de la matrice extracellulaire (Reed MJ et al., J. Cell Physiol, 1994, 158(1):169-79 ; Schiller M and al., J. Dermatol. Sci, 2004, 35(2):83-92).
- Le VEGF ou facteur de croissance endothélial vasculaire (en anglais « vascular endothelial growth factor ») qui représente dans la peau un facteur majeur de l'angiogénèse cutanée. L'épiderme est une source importante de VEGF, sécrété en grande quantité par les kératinocytes en prolifération. L'ARNm du VEGF est exprimé par les kératinocytes normaux, à la fois dans un tissu *in situ* et en culture cellulaire. Il a été montré que le VEGF maintiendrait l'homéostasie des cellules endothéliales et leur capacité à répondre à une stimulation angiogénique, même chez le sujet âgé (Watanabe Y et al., 1997, Oncogene 14 : 2025-2032). D'autre part une diminution du VEGF a été observée suite à une exposition aux radiations UV (Mildner M et al., Photochem. Photobiol., 1999; 70(4):674-9).
- Le HB-EGF ou facteur de croissance épidermique se liant à l'héparine (en anglais « heparin-binding epidermal growth factor »), qui joue un rôle important dans la régulation et la différenciation des kératinocytes (Iwamoto et al., Cytokine and Growth Factors Rewiews, 2000, 11:335-344), ainsi que dans la sénescence de cellules jeunes dont la croissance dépend de ce facteur (JID Suppl.24: S46-S50 ; Kanzaki Y et al., Exp. Cell. Res., 2002, 279(2):321-329).

L'invention décrit également l'utilisation d'un extrait de *Vanilla planifolia* selon l'invention en tant qu'agent activateur de la synthèse d'au moins un facteur de croissance cellulaire par les kératinocytes, notamment en tant qu'agent activateur d'au moins un facteur de croissance choisi parmi FGF-b, PDGF, TGFβ, VEGF et HB-EGF.

L'invention décrit également, l'utilisation d'un extrait de *Vanilla planifolia* selon l'invention en tant qu'agent inhibiteur de l'activité des métalloprotéinases de la matrice (dénommées MMP pour « matrix metalloprotease »).

Les métalloprotéinases de la matrice sont des enzymes qui dégradent la matrice extracellulaire dans le cadre du remodelage physiologique de la peau, mais l'âge et l'exposition aux irradiations UV ont pour conséquence d'augmenter l'activité de ces MMP, en particulier celle de la MMP1, de la MMP3 et de la MMP9. De ce fait, la matrice extracellulaire est dégradée de façon accrue, avec pour résultat l'affaissement des tissus de la peau et la formation de rides (Rittie L et al., Ageing Res. Rev., 2002, 1(4) :705-20 ; Chung JH et al., J. Invest. Dermatol, 2001, 117(5):1218-24).

Est également décrite, selon un aspect ultérieur, une composition cosmétique ou dermatologique comprenant un extrait de *Vanilla planifolia* tel que décrit plus haut et un véhicule cosmétiquement ou pharmaceutiquement acceptable.

De préférence, ledit extrait est présent dans la composition cosmétique ou dermatologique à raison de 0,001 à 10% en poids total de la composition, en particulier à raison de 0,01 à 10%, de préférence de 0,1 à 10% en poids total de la composition.

Ladite composition cosmétique ou dermatologique peut notamment, être adaptée à une application par voie topique.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau dans huile ou huile dans eau.

La composition cosmétique ou dermatologique selon l'invention comprend également un solvant choisi en fonction des différents ingrédients et de la forme d'administration.

A titre d'exemples, on peut citer l'eau (de préférence de l'eau déminéralisée), un alcool tel que l'éthanol, ou un éther de diéthylène glycol tel que l'éthoxydiglycol ou l'éther monométhylique de diéthylène glycol.

Ladite composition cosmétique peut également comprendre au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.

Notamment, ladite composition peut contenir :
- Un ou plusieurs agent(s) émollient(s) ou humectant(s), qui peuvent être choisi(s) par exemple parmi la glycérine, les glycols, les silicones hydrosolubles tels que celui vendu sous la dénomination KF6011 (Shin Etsu) et le Jojoba hydrosoluble, tel que celui vendu sous la dénomination Resplanta jojoba (Res pharma). Ledit agent émollient ou humectant peut être présent dans la composition à une teneur de l'ordre de 0 à 30%, de préférence 2 à 10% en poids, par rapport au poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghenanes, pectine), d'origine microbienne (xanthane), les argiles (laponite), les matériaux identifiés par les noms INCI "ammonium acryloyldiméthyltaurate/vp copolymer" et "ammonium acryloyldiméthyl-taurate/beheneth-25 méthacrylate copolymer" (tels que par exemple ceux vendus sous les dénominations Aristoflex AVC et HMB par Clariant).
   Ledit agent gélifiant et/ou épaississant peut être présent dans la composition à une teneur de l'ordre de 0 à 10% en poids, par rapport au poids total de la composition.
- Un ou plusieurs agent(s) tensioactif(s), de préférence non ionique, présent dans une teneur de l'ordre de 0 à 8%, de préférence 0,5 à 3% en poids, par rapport au poids total de la composition.
- Un ou plusieurs corps gras liquide(s) à température ambiante, communément dénommé(s) huiles(s), volatil(s) ou non volatile(s), hydrocarboné(s) ou siliconé(s), linéaire(s), cyclique(s) ou ramifié(s), par exemple, l'isododécane, le cyclopentadimethylsiloxane, les diméthicones, l'isononanoate d'isononyle, le pentaerythrityl tetraisostéarate, etc., de préférence à raison de 0 à environ 10%, de préférence 0,5 à 5% en poids, par rapport au poids total de la composition.
- Un ou plusieurs agent(s) actif(s) d'origine naturelle, biotechnologique ou synthétique ayant une activité biologique, par exemple choisis parmi les vitamines, les oligo éléments, l'allantoïne, les protéines végétales, les extraits végétaux, etc.
- Un ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle, de préférence à raison de 0 à environ 2% en poids, par rapport au poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention est illustrée de manière non limitative par les exemples ci-dessous.

### Exemple 1 : procédé de préparation d'un extrait selon l'invention

### 1) Extraction alcoolique

On broie 0,5 kg de gousses de *Vanilla planifolia* à l'aide d'un broyeur à couteaux (RETSCH) que l'on charge dans un réacteur en verre de 5 l équipé d'un reflux.

On ajoute 2,5 l d'éthanol à 96,3% et on chauffe pendant une heure. On laisse macérer à froid pendant une nuit.

Au matin, on filtre et on vidange le réacteur.

Le solvant chargé est conservé. Le broyat de vanille est de nouveau chargé dans le réacteur avec 2,5 l d'éthanol à 96,3 %. On chauffe pendant 4 h au reflux à environ 80°C.

On filtre et on vidange le réacteur. Les deux filtrats sont rassemblés.

On évapore ensuite le solvant avec un évaporateur rotatif sous vide.

On récupère ainsi 0,155 kg d'oléorésine de *Vanilla planifolia.*

Rendement : 31 %.

### 2) Reprise hydro alcoolique

On prépare de l'éthanol aqueux par mélange à froid d'éthanol 96,3 % et d'eau (70/30 p/p).

L'oléorésine de vanille obtenue à l'étape précédente est fondue à 60° C pour faciliter sa dilution.

On mélange ensuite 8 kg d'oléorésine avec 2 kg d'éthanol aqueux et on mélange avec l'homogénéisateur jusqu'à homogénéité totale.

Après mise en décantation du mélange pendant 72 h, il y a apparition d'un surnageant liposoluble à la partie supérieure.

On soutire la phase inférieure et on récupère alors la phase supérieure, puis on évapore le solvant de la dite solution sous vide avec un évaporateur rotatif.

On obtient ainsi 2,38 kg de fraction liposoluble de *Vanilla planifolia.*

Le rendement de cette opération est de 29,8 %.

### 3) Distillation moléculaire

150 g de la fraction liposoluble de *Vanilla planifolia* obtenue lors de l'étape précédente sont mélangés à 50 g de polyéthylèneglycol 600 (Dénomination INCI : PEG12) :

On effectue deux passages dans un appareil de distillation de type KDL4 (UIC GmH) selon les paramètres de distillation suivants :

| **Paramètres de distillation** | ***1er passage*** | ***2nd passage*** |
|---|---|---|
| Débit d'insertion (ml/h) | 400 | 400 |
| Température évaporateur (°C) | 185 | 190 |
| Température condenseur (°C) | 75 | 75 |
| Température d'introduction du produit (°C) | 75 | 75 |
| Agitation (tours/min) | 180 | 180 |
| Pression vide (mbar) | 2,2.10⁻² à 9,0.10⁻³ | 2,2.10⁻² à 9,0.10⁻³ |

Les rendements de la distillation sont les suivants, exprimés sur le poids de manière première :
Distillat 1^{er} passage = 18,53 %
Distillat 2^{nd} passage = 8,67 %

### 4) Centrifugation

On récupère le distillat 1^{er} passage, que l'on laisse décanter, puis on prélève le surnageant, que l'on centrifuge et à environ 4000 tours/min pendant 10 min, et on récupère la phase supérieure chargée en huile.

Le culot de décantation est également passé à la centrifugeuse et on récupère la phase supérieure chargée en huile.

Après rassemblement des différentes huiles, on obtient le distillat 1^{er} passage final, soit 24,22 g d'huile.

Le distillat 2^{nd} passage ne contient pas de gomme, il ne subit donc pas cette étape de centrifugation.

On rassemble toutes les fractions huileuses, c'est-à-dire les distillats du 1^{er} passage et du 2^{nd} passage, soit 24,22 g + 13 g = 37,22 g

Le rendement de la distillation moléculaire et de la centrifugation est de 24,81 %.

### Exemple 2 : analyse de la composition de l'extrait de Vanilla planifolia selon l'invention

1) 100 mg d'extrait de *Vanilla planifolia* obtenus comme décrit dans l'exemple 1 sont solubilisés dans 2 ml d'une solution méthanolique d'acide chlorhydrique 1N. La solution est placée après agitation au bain-marie à 80°C pendant 10 min.

L'échantillon estérifié est récupéré par extraction liquide/liquide avec 2 ml d'heptane et injecté dans le chromatographe.

La chromatographie est réalisée dans les conditions suivantes :
Chromatographe en phase gazeuse (Agilent série 6890).
Injecteur avec ou sans division à 250°C.

Injection de 0,5µl d'échantillon préparé avec une division de 1/100^{ème}.

Séparation sur colonne capillaire 100 % polydiméthylsiloxane greffée, longueur 30 m, diamètre interne 0,25 mm, épaisseur de film : 0,25µm (Agilent série DB-1)

Gaz vecteur : Hélium à 1ml/min

Température initiale du four colonne : 70°C

Gradient de température de 70°C à 250°C à 2°C/min et isotherme à 250°C pendant 60 min.

Durée totale : 150 min.

Détection : détecteur à ionisation de flamme (gaz auxiliaire azote).

Les pourcentages donnés dans le tableau 1 ci-dessous sont exprimés en pourcentages relatifs obtenus par rapport à l'ensemble des constituants séparés par le système chromatographique. Seuls les composés extraits par l'hexane lors de la préparation de l'échantillon et seuls les composés pouvant se vaporiser dans l'injecteur sont présents dans ces résultats.

L'identification des constituants a été réalisée par spectrométrie de masse.

Les résultats sont donnés dans le tableau 1 ci-dessous.

**Tableau 1**

| **T_{R}** | **Constituants** | **%GC relatifs** |
|---|---|---|
| 5,3 | Nonane | 0,10 |
| 34,3 | Myristate de méthyle | 0,09 |
| 37,7 | Pentadécanoate de méthyle | 0,16 |
| 41,0 | Palmitate de méthyle | 2,35 |
| 44,1 | Heptadécanoate de méthyle | 0,20 |
| 45,9 | Linoléate de méthyle | 6,42 |
| 46,0 | Linolénate de méthyle | 1,12 |
| 46,2 | Oléate de méthyle | 0,99 |
| 46,7 | Hénéicosane | 0,09 |
| 47,1 | Stéarate de méthyle | 0,52 |
| 49,5 | Docosane | 0,09 |
| 51,5 | Tricosène | 0,33 |
| 52,4 | Tricosane | 0,38 |
| 52,7 | Eicosanoate de méthyle | 0,11 |
| 55,0 | Tétracosane | 0,22 |
| 56,8 | Pentacosène | 0,29 |
| 57,1 | Docosén-13-oate de méthyle | 0,17 |
| 57,5 | Pentacosane | 0,45 |
| 57,9 | Docosanoate de méthyle | 0,15 |
| 59,6 | Tricosénoate de méthyle | 0,17 |
| 60,3 | Hexacosane | 0,10 |
| 60,8 | Tricosanoate de méthyle | 0,10 |
| 61,7 | Pentacosèn-16-one-2 | 0,51 |
| 62,2 | Tétracosén-15-oate de méthyle | 2,46 |
| 62,6 | Heptacosane | 0,41 |
| 63,0 | Tétracosanoate de méthyle | 0,29 |
| 64,5 | Pentacosène-16-dione-2,4 | 1,66 |
| 64,9 | Pentacosénoate de méthyle | 0,52 |
| 65,9 | Octacosane | 0,11 |
| 66,6 | Pentacosanoate de méthyle | 0,15 |
| 68,5 | Heptacosèn-18-one-2 | 0,47 |
| 69,0 | Nonadécèn-10-yl-2 dihydro-2,3 méthyl-6 4H-Pyranone-4 | 0,64 |
| 69,3 | Hexacosénoate de méthyle | 0,74 |
| 70,3 | Nonacosane | 0,07 |
| 70,5 | Hexacosanoate de méthyle | 0,10 |
| 70,8 | Heptacosénoate de méthyle | 1,17 |
| 72,7 | Heptacosanoate de méthyle | 0,22 |
| 73,1 | Heptacosène-18-dione-2,4 (nervonoylacétone) | 38,14 |
| 76,8 | Triacontane | 2,33 |
| 79,4 | Octacosénoate de méthyle | 0,28 |
| 79,9 | Nonacosèn-20-one-2 | 0,96 |
| 80,4 | Hénéicosèn-12-yl-2 dihydro-2,3 méthyl-6 4H-Pyranone-4 | 2,68 |
| 81,0 | Octacosanoate de méthyle | 0,60 |
| 82,4 | Hentriacontane | 0,21 |
| 86,7 | Nonacosène-20-dione-2,4 | 9,59 |
| 92,5 | Triaconténoate de méthyle | 1,05 |
| 98,9 | Tricosèn-14-yl-2 dihydro-2,3 méthyl-6 4H-Pyranone-4 | 6,47 |
| 109,0 | Hentriacontène-22-dione-2,4 | 3,04 |
| | **Total % GC** | **89,47** |

### Exemple 3 : étude de l'activité d'extraits de Vanilla planifolia selon l'invention vis-à-vis du facteur de croissance HB-EGF

On a testé, d'une part, un extrait de *Vanilla planifolia* obtenu dans l'exemple 1, à la fin de l'étape 2, dénommé ci après « fraction liposoluble » et, d'autre part, un extrait de *Vanilla planifolia* obtenu à la fin de l'étape 4, dénommé ci-après « distillat huileux».

### 1) Préparation des cultures de kératinocytes

Ce protocole est commun à tous les essais d'activité biologique.

Des kératinocytes dérivés de prépuces néonataux (Clonetics, San Diego, USA) ont été ensemencés dans des plaques à 6 puits et cultivés dans du milieu de culture pour la croissance des kératinocytes (KBM, Clonetics), à savoir un milieu de culture modifié supplémenté en EGF humain recombinant, insuline, hydrocortisone, extrait hypophysaire bovin, gentamycine et amphotéricine b.

Après 24 h de culture dans une étuve à 37°C, les cellules confluentes ont été lavées avec du tampon PBS (Gibco) et incubées avec du milieu spécifique basique (KBM, Clonetics) contenant les produits à tester, pendant 24 h, à des concentrations de 500, 10, 1 ou 0,1 µg/ml. Après étude de la cytotoxicité de l'extrait, son activité a été évaluée. Toutes les concentrations n'ont pas nécessairement été testées dans tous les essais.

### 2) Mesure de l'expression de l'ARN messager (ARNm) du HB-EGF

### Principe de l'essai :

On utilise l'amplification en chaîne par polymérase en temps réel (en anglais RT-PCR pour « real time-polymerase chain reaction ») pour quantifier l'expression de l'ARN messager du HB-EGF dans un échantillon traité par rapport à un échantillon non traité. Les résultats sont normalisés par rapport à l'expression de gènes domestiques dans ces échantillons et corrigés en ce qui concerne les différences d'efficacité de PCR.

Les résultats sont exprimés en nombre de fois d'augmentation ou de diminution d'expression du gène cible (HB-EGF) dans l'échantillon traité, et non en nombre absolu de copies.

Les séquences des ADNc/ARNm des gènes investigués ont été obtenues chez GenBank.

Gène cible : HB-EGF

Gène domestique :PBDG

Toutes les amorces de PCR ont été obtenues en utilisant le logiciel PRIMER3 du Whitehead Institute for Biomédical Resarch.

### Protocole de l'essai :

L'ARNm a été isolé en utilisant le réactif Trizol (Invitrogen) selon les recommandations du fabricant. La transcription inverse a été effectuée à l'aide du kit gene Amp RNA PCR (Applied Biosystems) selon les recommandations du fabricant.

La mesure de PCR en temps réel a été effectuée en utilisant l'appareil iCYCLER IQ (Biorad) avec détection SYBR Green I. Dans tous les essais, l'ADNc a été amplifié en utilisant un programme standardisé. Chaque échantillon a été chargé avec du supermixe IQ SYBR Green I, de l'eau et de l'amorce (stock) ; la quantité finale d'ADNc par réaction correspondait à 25 ng d'ARN total utilisé pour la transcription inverse.

La spécificité de la PCR a été testée par électrophorèse sur gel d'agarose et évaluée pour chaque échantillon en utilsant une analyse de point de fusion incluse dans le programme de PCR.

La quantification relative de l'expression du gène cible a été réalisée en utilisant le modèle mathématique de Pfaffl (Pfaffl, MW, Nucleic Acids Res. 29(9), p. E45, 2001).

Les résultats sont rapportés dans le tableau 2 ci-dessous :

**Tableau 2**

| **Produit testé** | **Concentration (µg /ml)** | **Activité (stimulation)** |
|---|---|---|
| Fraction liposoluble | 10 | non détectable |
| | 100 | 3,1 |
| | 500 | 12,6 |
| Distillat huileux | 10 | 2 |
| | 100 | 4,7 |
| | 500 | 16,4 |

Les résultats montrent que les extraits de *Vanilla planifolia* selon l'invention possèdent une activité stimulatrice de la synthèse du facteur de croissance HB-EGF.

### Exemple 4 : étude de l'activité d'extraits de Vanilla planifolia selon l'invention vis-à-vis du facteur de croissance TGF-β1

L'évaluation quantitative des concentrations du facteur de croissance TGF-β1 activé dans les cultures de cellules a été effectuée à l'aide de la méthode ELISA en utilisant le kit d'immunoessai Quantikine^{®} (n° DB100, R&D Systems) .

Les conditions de culture des kératinocytes et les échantillons testés sont tels que décrits dans l'exemple 3.

Les résultats sont rapportés dans le tableau 3 ci-dessous :

**Tableau 3**

| **Produit testé** | **Concentration (µg /ml)** | **Activité (stimulation)** |
|---|---|---|
| Fraction liposoluble | 1 | 100% |
| | 10 | 179% |
| Distillat huileux | 1 | 132,6% |
| | 10 | 121% |

Les résultats montrent que les extraits de *Vanilla planifolia* selon l'invention possèdent une activité stimulatrice de la synthèse du facteur de croissance TGF-β1.

### Exemple 5 : étude de l'activité d'extraits de Vanilla planifolia selon l'invention vis-à-vis du facteur de croissance VEGF

L'évaluation quantitative des concentrations du facteur de croissance VEGF dans les cultures de cellules a été effectuée à l'aide de la méthode ELISA en utilisant le kit d'immunoessai Quantikine^{®} (n° DVE00, R&D Systems) .

Les conditions de culture des kératinocytes et les échantillons testés sont tels que décrits dans l'exemple 3.

Les résultats sont rapportés dans le tableau 4 ci-dessous :

**Tableau 4**

| **Produit testé** | **Concentration (µg /ml)** | **Activité (stimulation)** |
|---|---|---|
| Fraction liposoluble | 0,1 | 168,5% |
| | 1 | 179,4% |
| | 10 | 155,8% |
| | 100 | 200% |
| | 500 | 200,3% |
| Distillat huileux | 0,1 | 145,9% |
| | 1 | 158,1% |
| | 10 | 170,6% |
| | 100 | 187,8% |
| | 500 | 198,1% |

Les résultats montrent que les extraits de *Vanilla planifolia* selon l'invention possèdent une activité stimulatrice de la synthèse du facteur de croissance VEGF.

### Exemple 6 : étude de l'activité d'extraits de Vanilla planifolia selon l'invention vis-à-vis du facteur de croissance PDGF-AA

L'évaluation quantitative des concentrations du facteur de croissance PDGF-AA humain dans les cultures de cellules a été effectuée à l'aide de la méthode ELISA en utilisant le kit d'immunoessai Quantikine^{®} (n° DATA00, R&D Systems) .

Les conditions de culture des kératinocytes et les échantillons testés sont tels que décrits dans l'exemple 3.

Les résultats sont rapportés dans le tableau 5 ci-dessous :

**Tableau 5**

| **Produit testé** | **Concentration (µg /ml)** | **Activité (stimulation)** |
|---|---|---|
| Fraction liposoluble | 0,1 | 133,8% |
| | 1 | 131,8% |
| | 10 | 137,0% |
| Distillat huileux | 0,1 | 137,1% |
| | 1 | 131,8% |
| | 10 | 145,8% |

Les résultats montrent que les extraits de *Vanilla planifolia* selon l'invention possèdent une activité stimulatrice de la synthèse du facteur de croissance PDGF-AA .

### Exemple 7 : étude de l'activité d'extraits de Vanilla planifolia selon l'invention vis-à-vis du facteur de croissance FGF-b

L'évaluation quantitative des concentrations du facteur de croissance FGF-b humain dans les cultures de cellules a été effectuée à l'aide de la méthode ELISA en utilisant le kit d'immunoessai Quantikine^{®} (n° DFB50, R&D Systems) .

Les conditions de culture des kératinocytes et les échantillons testés sont tels que décrits dans l'exemple 3.

Les résultats sont rapportés dans le tableau 6 ci-dessous :

**Tableau 6**

| **Produit testé** | **Concentration (µg /ml)** | **Activité (stimulation)** |
|---|---|---|
| Fraction liposoluble | 10 | 100% |
| | 100 | 321,1% |
| | 500 | 433,3% |
| Distillat huileux | 10 | 484,8% |
| | 100 | 175,8% |
| | 500 | 145,8% |

Les résultats montrent que les extraits de *Vanilla planifolia* selon l'invention possèdent une activité stimulatrice de la synthèse du facteur de croissance FGF-b .

### Exemple 8 : étude de l'activité d'extraits de Vanilla planifolia selon l'invention vis-à-vis de l'endothéline-1

L'évaluation quantitative des concentrations d'endothéline-1 humaine dans les cultures de cellules a été effectuée à l'aide de la méthode ELISA en utilisant le kit d'immunoessai Quantikine^{®} (n° BBE5, R&D Systems) .

Les conditions de culture des kératinocytes et les échantillons testés sont tels que décrits dans l'exemple 3.

Les résultats sont rapportés dans le tableau 7 ci-dessous :

**Tableau 7**

| **Produit testé** | **Concentration (µg /ml)** | **Activité (inhibition)** |
|---|---|---|
| Fraction liposoluble | 10 | 0% |
| | 100 | 75,7% |
| | 500 | 72,2% |
| Distillat huileux | 10 | 27,8% |
| | 100 | 82,9% |
| | 500 | 94,9% |

Les résultats montrent que les extraits de *Vanilla planifolia* selon l'invention possèdent une activité inhibitrice de la synthèse d'endothéline-1.

### Exemple 9 : étude de l'activité d'extraits de Vanilla planifolia selon l'invention vis-à-vis des métalloprotéinases de la matrice (MMP)

### Principe de l'essai

Les produits à tester sont incubés avec les MMP activées L'activité enzymatique est contrôlée par l'addition d'un substrat enzymatique fluorescent spécifique de chaque MMP.

### Protocole de l'essai

La pro-MMP1, la pro-MMP2 ou la pro-MMP9 sont incubées dans les plaques avec une solution d'APMA (acétate p-aminophénylmercurique) à température ambiante, sous légère agitation, pendant 1 h.

Différentes concentrations des différents inhibiteurs potentiels sont ajoutées. On incube ensuite le mélange à température ambiante sous légère agitation. La réaction enzymatique est provoquée en ajoutant le substrat fluorescent solubilisé dans du DMSO.

La réaction enzymatique est suivie pendant 1 h à l'aide d'un spectrofluorimètre et la fluorescence est mesurée à une longueur d'onde d'excitation de 360 nm et une longueur d'onde d'émission de 460 nm pour MMP1 ; à une longueur d'onde d'excitation de 320 nm et une longueur d'onde d'émission de 405 nm pour MMP2 et MMP9.

Les résultats sont exprimés en unité de fluorescence émise (RFU) qui représente la quantité de substrat hydrolysé par min.

Le spectrofluorimètre Microwin 2000 calcule la variation d'absorbance Δ, qui représente la vitesse initiale (Vi) de la réaction enzymatique. Dans chaque essai, on fait la moyenne des 3 valeurs de Vi obtenues pour chaque concentration d'inhibiteur potentiel. Les résultats de 10 expériences ont été exprimés en moyenne ± SD, puis présentés en pourcentage d'activité résiduelle.

Les résultats sont rapportés dans les tableaux 8-10 ci-dessous :

**Tableau 8**

| **Produit testé** | **Concentration (µg /ml)** | **Activité (inhibition) sur MMP1** |
|---|---|---|
| Fraction liposoluble | 100 | 53% |
| | 500 | 94% |
| | 1000 | 99% |
| Distillat huileux | 100 | 27% |
| | 500 | 72% |
| | 1000 | 91% |

Les résultats montrent que les extraits de *Vanilla planifolia* selon l'invention possèdent une activité inhibitrice des MMP1.

**Tableau 9**

| **Produit testé** | **Concentration (µg /ml)** | **Activité (inhibition) sur MMP2** |
|---|---|---|
| Fraction liposoluble | 10 | 0% |
| | 100 | 37% |
| | 500 | 88% |
| | 1000 | 94% |
| Distillat huileux | 10 | 21% |
| | 100 | 33% |
| | 500 | 62% |
| | 1000 | 78% |

Les résultats montrent que les extraits de *Vanilla planifolia* selon l'invention possèdent une activité inhibitrice des MMP2.

**Tableau 10**

| **Produit testé** | **Concentration (µg /ml)** | **Activité (inhibition) sur MMP9** |
|---|---|---|
| Fraction liposoluble | 100 | 536% |
| | 500 | 84% |
| | 1000 | 96% |
| Distillat huileux | 100 | 23% |
| | 500 | 60% |
| | 1000 | 75% |

Les résultats montrent que les extraits de *Vanilla planifolia* selon l'invention possèdent une activité inhibitrice des MMP9.

### Exemple 10 : Emulsions huile-dans-eau (H/E)

Les compositions suivantes peuvent être préparées de façon classique pour l'homme du métier. Les quantités indiquées ci-dessous sont exprimées en pourcentages pondéraux.

| Emulsion A | |
|---|---|
| Cetearyl alcohol & cetearyl glucoside | 4.00 % |
| Beheneth-25 | 2.00 % |
| Fraction liposoluble de *Vanilla planifolia** | 1.00 % |
| Licorice extract | 0.10 % |
| Emollients | 35.00 % |
| Tocopheryl acetate | 0.50 % |
| Dimethicone | 2.00 % |
| EDTA | 0.05 % |
| Glycérine | 5.00 % |
| Gélifiants | 2.00 % |
| Ajusteur de pH | qs |
| Conservateurs | qs |
| Eau | qsp 100.00 % |

| | |
|---|---|
| * préparée comme décrit à l'Exemple 1 | |

| Emulsion B | |
|---|---|
| Beheneth-25 | 2.00 % |
| Distillat huileux de *Vanilla planifolia** | 0.10 % |
| Hydrocotyl extract | 0.20 % |
| Tenseur | 5.00 % |
| Emollients | 15.00 % |
| Filtres UV | 0.50 % |
| Tocopheryl acetate | 0.50 % |
| Cyclomethicone | 5.00 % |
| EDTA | 0.05 % |
| Glycérine | 5.00 % |
| Gélifiants | 2.00 % |
| Ajusteur de pH | qs |
| Conservateurs | qs |
| Eau | qsp 100.00 % |

| | |
|---|---|
| * préparé comme décrit à l'Exemple 1 | |

## Revendications

1. Extrait de *Vanilla planifolia,* susceptible d'être obtenu suivant un procédé comprenant les étapes consistant à :
- procéder à une extraction à partir d'un broyat de *Vanilla planifolia* à l'aide d'au moins un solvant,
- récupérer la fraction liposoluble,
- et comprenant en outre une étape de distillation moléculaire de la fraction liposoluble pour obtenir un distillat huileux, ladite distillation moléculaire étant effectuée à une température comprise entre 100 et 250°C et une pression comprise entre 0,1 et 0,001 mbar, et
- récupérer le distillat.

2. Extrait selon la revendication 1, **caractérisé en ce que** ladite fraction liposoluble comprend :
- 0,5 à 10% de composés monocarbonylés insaturés,
- 20 à 80% de composés dicarbonylés insaturés, et
- 1 à 40% de pyranones insaturées,
lesdites proportions étant exprimées en pourcentages relatifs par rapport à l'ensemble des constituants séparés par chromatographie en phase gazeuse.

3. Extrait selon l'une des revendications 1 ou 2, **caractérisé en ce que** ladite fraction comprend :
- 1 à 5% de composés monocarbonylés insaturés,
- 35 à 65% de composés dicarbonylés insaturés, et
- 3 à 25% de pyranones insaturées,
lesdites proportions étant exprimées en pourcentages relatifs par rapport à l'ensemble des constituants séparés par chromatographie en phase gazeuse.

4. Extrait selon l'une quelconque des revendications 1 à 3, **caractérisé en ce qu'**il comprend des composés monocarbonylés insaturés choisis parmi la Pentacosén-16-one-2, la Heptacosén-18-one-2 et la Nonacosén-20-one-2.

5. Extrait selon l'une quelconque des revendications 1 à 4, **caractérisé en ce qu'**il comprend des composés dicarbonylés insaturés choisis parmi la Pentacosène-16-dione-2,4, la Heptacosène-18-dione-2,4, la Nonacosène-20-dione-2,4 et l'Hentriacontène-22-dione-2,4.

6. Extrait selon l'une quelconque des revendications 1 à 5, **caractérisé en ce qu'**il comprend des pyranones insaturées choisies parmi la nonadécényl-2 dihydro-2,3 méthyl-6 pyranone-4, l'hénéicosényl-2 dihydro-2,3 méthyl-6 pyranone-4 et la tricosényl-2 dihydro-2,3 méthyl-6 pyranone-4.

7. Extrait selon l'une quelconque des revendications 1 à 6, **caractérisé en ce que** ladite fraction comprend, en outre, de 5 à 50% d'acides gras saturés ou insaturés, lesdites proportions étant exprimées en pourcentages relatifs par rapport à l'ensemble des constituants séparés par chromatographie en phase gazeuse.

8. Extrait selon l'une quelconque des revendications 1 à 7, **caractérisé en ce qu'**il comprend des composés stérols choisis parmi le cholestérol, le campestérol, le stigmastérol et le β-sitostérol.

9. Utilisation d'un extrait de *Vanilla planifolia* selon l'une quelconque des revendications 1 à 8 pour la prévention et/ou le traitement d'altérations de la peau dues au vieillissement.

10. Utilisation d'un extrait de *Vanilla planifolia* selon l'une quelconque des revendications 1 à 8 comme agent dépigmentant de la peau.

## Patentansprüche

1. Extrakt aus *Vanilla planifolia,* erhältlich durch ein Verfahren, welches die Schritte umfasst, bestehend aus:
- Durchführen einer Extraktion, ausgehend von Häckselgut von *Vanilla planifolia,* unter Verwendung mindestens eines Lösungsmittels,
- Gewinnen der fettlöslichen Fraktion,
- und ferner umfassend einen Schritt der Molekulardestillation der fettlöslichen Fraktion, um ein öliges Destillat zu erhalten, wobei die Molekulardestillation bei einer Temperatur zwischen 100 und 250°C und einem Druck zwischen 0,1 und 0,001 mbar durchgeführt wird, und
- Gewinnen des Destillats.

2. Extrakt nach Anspruch 1, **dadurch gekennzeichnet, dass** die fettlösliche Fraktion:
- 0,5 bis 10 % ungesättigte Monocarbonylverbindungen,
- 20 bis 80 % ungesättigte Dicarbonylverbindungen und
- 1 bis 40 % ungesättigte Pyranone,
umfasst,
wobei die Anteile durch Prozent in Bezug auf die Gesamtheit der durch Gasphasenchromatographie getrennten Bestandteile ausgedrückt sind.

3. Extrakt nach einem der Ansprüche 1 oder 2, **dadurch gekennzeichnet, dass** die Fraktion:
- 1 bis 5 % ungesättigte Monocarbonylverbindungen,
- 35 bis 65 % ungesättigte Dicarbonylverbindungen und
- 3 bis 25 % ungesättigte Pyranone,
umfasst,
wobei die Anteile durch Prozent in Bezug auf die Gesamtheit der durch Gasphasenchromatographie getrennten Bestandteile ausgedrückt sind.

4. Extrakt nach einem der Ansprüche 1 bis 3, **dadurch gekennzeichnet, dass** es ungesättigte Monocarbonylverbindungen umfasst, ausgewählt aus Pentacosen-16-on-2, Heptacosen-18-on-2 und Nonacosen-20-on-2.

5. Extrakt nach einem der Ansprüche 1 bis 4, **dadurch gekennzeichnet, dass** es ungesättigte Dicarbonylverbindungen umfasst, ausgewählt aus Pentacosen-16-dion-2,4, Heptacosen-18-dion-2,4, Nonacosen-20-dion-2,4 und Hentriaconten-22-dion-2,4.

6. Extrakt nach einem der Ansprüche 1 bis 5, **dadurch gekennzeichnet, dass** es ungesättigte Pyranone umfasst, ausgewählt aus Nonadecenyl-2 dihydro-2,3 methyl-6 pyranon-4, Heneicosenyl-2 dihydro-2,3 methyl-6 pyranon-4 und Tricosenyl-2 dihydro-2,3 methyl-6 pyranon-4.

7. Extrakt nach einem der Ansprüche 1 bis 6, **dadurch gekennzeichnet, dass** die Fraktion ferner von 5 bis 50% gesättigte oder ungesättigte Fettsäuren umfasst,
wobei die Anteile durch Prozent in Bezug auf die Gesamtheit der durch Gasphasenchromatographie getrennten Bestandteile ausgedrückt sind.

8. Extrakt nach einem der Ansprüche 1 bis 7, **dadurch gekennzeichnet, dass** es Sterolverbindungen umfasst, ausgewählt aus Cholesterol, Campesterol, Stigmasterol und β-Sitosterol.

9. Verwendung eines Extrakts aus *Vanilla planifolia* nach einem der Ansprüche 1 bis 8 zur Prävention und/oder Behandlung von Hautveränderungen aufgrund von Alterung.

10. Verwendung eines Extrakts aus *Vanilla planifolia* nach einem der Ansprüche 1 bis 8 als ein Depigmentierungsmittel der Haut.

## Claims

1. Extract of *Vanilla planifolia* obtainable according to a method comprising the steps consisting of:
- performing an extraction from a mash of *Vanilla planifolia* by means of at least one solvent,
- recovering the fat-soluble fraction,
- and further comprising a step of molecular distillation of the fat-soluble fraction to obtain an oily distillate, said molecular distillation being performed at a temperature of between 100 and 250°C and under a pressure of between 0.1 and 0.001 mbar, and
- recovering the distillate.

2. Extract according to claim 1, **characterised in that** said fat-soluble fraction comprises:
- 0.5 to 10% unsaturated monocarbonylated compounds,
- 20 to 80% unsaturated dicarbonylated compounds, and
- 1 to 40% unsaturated pyranones,
said proportions being expressed in relative percentages with respect to the total of all of the constituents separated by chromatography in a gaseous phase.

3. Extract according to either claim 1 or claim 2, **characterised in that** said fraction comprises:
- 1 to 5% unsaturated monocarbonylated compounds,
- 35 to 65% unsaturated dicarbonylated compounds, and
- 3 to 25% unsaturated pyranones,
said proportions being expressed in relative percentages with respect to the total of all of the constituents separated by chromatography in a gaseous phase.

4. Extract according to any one of claims 1 to 3, **characterised in that** it comprises unsaturated monocarbonylated compounds selected from Pentacosene-16-one-2, Heptacosene-18-one-2 and Nonacosene-20-one-2.

5. Extract according to any one of claims 1 to 4, **characterised in that** it comprises unsaturated dicarbonylated compounds selected from Pentacosene-16-dione-2,4, Heptacosene-18-dione-2,4, Nonacosene-20-dione-2,4 and Hentriacontene-22-dione-2,4.

6. Extract according to any one of claims 1 to 5, **characterised in that** it comprises unsaturated pyranones selected from nonadecenyl-2 dihydro-2,3 methyl-6 pyranone-4, heneicosenyl-2 dihydro-2,3 methyl-6 pyranone-4 and tricosenyl-2 dihydro-2,3 methyl-6 pyranone-4.

7. Extract according to any one of claims 1 to 6, **characterised in that** said fraction further comprises 5 to 50% of saturated or unsaturated fatty acids, said proportions being expressed in relative percentages with respect to the total of all of the constituents separated by chromatography in a gaseous phase.

8. Extract according to any one of claims 1 to 7, **characterised in that** it comprises sterol compounds selected from cholesterol, campesterol, stigmasterol and β-sitosterol.

9. Use of an extract of *Vanilla planifolia* according to any one of claims 1 to 8 for the prevention and/or the treatment of skin alterations caused by ageing.

10. Use of an extract of *Vanilla planifolia* according to any one of claims 1 to 8 as a skin depigmentation agent.
